# EUROPÄISCHE PATENTSCHRIFT

(11) **EP 4 103 274 B1**
(45) Veröffentlichungstag und Bekanntmachung des Hinweises auf die Patenterteilung: **18.11.2026**
(21) Anmeldenummer: 21705470.9
(22) Anmeldetag: 11.02.2021
(51) Int. Cl.: A61N 5/06, A61M 25/00

(54) **TRANSURETHRALER BLASENKATHETER MIT ANTIMIKROBIELLER WIRKUNG ZUR VERHINDERUNG DER ADHÄSION**
TRANSURETHRAL BLADDER CATHETER WITH ANTIMICROBIAL EFFECT TO PREVENT ADHESION
CATHÉTER VÉSICAL TRANSURETHRAL À L'EFFET ANTIMICROBIEN POUR ÉVITER L'ADHÉSION

(30) Priorität: 14.02.2020 DE 102020000934
(43) Veröffentlichungstag der Anmeldung: 21.12.2022
(73) Patentinhaber: Albert-Ludwigs-Universität Freiburg, 79098 Freiburg (DE)
(72) Erfinder: AL AHMAD, Ali, 79117 Freiburg (DE); HELLWIG, Elmar, 79102 Freiburg (DE); HILLER, Joachim, 73035 Göppingen (DE); KATZ, Till, 70186 Stuttgart (DE)
(74) Vertreter: Hertin und Partner Rechts- und Patentanwälte PartG mbB
(86) Internationale Anmeldenummer: PCT/EP2021/053360
(87) Internationale Veröffentlichungsnummer: WO 2021/160751

(56) Entgegenhaltungen:
- WO-A2-2019/088940
- CN-Y- 201 135 707
- US-A- 5 188 593
- US-A1- 2018 369 603

## Beschreibung

### Hintergrund der Erfindung

Die Erfindung betrifft einen transurethralen Blasenkatheter. Blasenkatheter, auch Urin- oder Urinalkatheter genannt, werden in der Medizin sowohl aus therapeutischen Gründen als auch zu diagnostischen Zwecken eingesetzt. Grundsätzlich dienen sie sowohl der Harnableitung, wenn der Patient nicht mehr eigenständig Wasser lassen kann, beispielsweise bei einer Vergrößerung der Prostata, einer neurogenen Blasenentleerungsstörung, einem durch Medikamente verursachten Harnverhalt oder einer Entzündung der Harnröhre oder Blase, als auch der Harngewinnung, zur Untersuchung des Urins auf Keime oder der Beobachtung seines zeitlichen Abflusses im Hinblick auf dessen Menge und Konzentration zur Erkennung von Nierenfunktionsstörungen. Außerdem werden sie zum Spülen der Harnblase oder zum Einbringen von Medikamenten verwendet und kommen bei der Kontrastmitteleingabe für bildgebende Verfahren im Harnwegebereich zum Einsatz.

Ein transurethraler Blasenkatheter wird über die Harnröhre (transurethral) in die Harnblase eingebracht. Dazu besteht er im Wesentlichen aus einem Schlauch aus Kunststoff, dessen Lumen der Urinableitung dient. Man unterscheidet in transurethrale Einmal- und Dauerkatheter, wobei letztere zur längerfristigen Benutzung eingesetzt werden und einen zweiten Lumen (man spricht auch von Nebenlumen gegenüber dem erstgenannten Hauptlumen) aufweisen, der zum Füllen, z.B. mit sterilem destillierten Wasser oder einer speziellen 10-%-Glycerinlösung, eines an der Spitze des Katheters zusätzlich angebrachten manschettenförmigen Ballons vorgesehen ist, sodass der Katheter im aufgeblasenen Zustand in der Blase verbleibt bzw. nicht mehr herausrutschen kann (blockiert). Der Nebenlumen ist dabei mit einem Ventil ausgestattet, um ein Entweichen des Blockmediums zu verhindern.

Die letztgenannten, zur dauerhaften Harnableitung eingesetzten transurethralen Blasenkatheter werden wegen ihres Haupt- und Nebenlumen auch 2-Wege Katheter genannt. Insbesondere bei starken Blutungen in der Blase oder nach transurethralen Operationen kommen in der Urologie auch 3-Wege-Katheter zum Einsatz. Neben den oben genannten beiden Lumina besitzen diese noch einen weiteren Nebenlumen, der zur Einbringung von Spüllösungen genutzt werden kann, um eine Gerinnung des Blutes in der Harnblase zu verhindern. Weiterhin existieren bereits mehrlumige transurethrale Blasenkatheter, deren zusätzliche Nebenlumen urologische Spezialuntersuchungen (z.B. Blasendruckmessungen) ermöglichen oder speziellen Therapien (z.B. photodynamischen Therapien (PdT)) dienen.

Allen transurethralen Blasenkathetern ist gemeinsam, dass ihre Hauptlumen an ihrem distalen Ende jeweils mit einem Urinbeutel zum Auffangen des Urins verbunden sind. Die Urinbeutel selbst besitzen ebenfalls Einwegventile, die gewährleisten, dass ein Herausfließen des Urins beim Beutelwechsel verhindert wird.

### Stand der Technik

Aus der DE 694 10 123 T2 sind transurethrale 2-Wege- und 3-Wege-Urinkatheter der aufgeführten Art bekannt. Neben der üblichen Nutzung ihrer Lumina (Harnableitung, Ballonfüllung, Spülung) sind diese darauf gerichtet, eine Reduzierung des Querschnitts des Hauptlumens durch besonders geformte Rippen zu vermeiden, um die Durchflussrate des Katheters nicht zu beeinträchtigen. Eine Möglichkeit zum Durchführen einer PdT, wie es beim Gegenstand der vorliegenden Erfindung der Fall ist, ist nicht gegeben. Von einem derartigen, in seinem Grundaufbau allgemein bekannten, herkömmlichen transurethralen Blasenkatheter wird stattdessen beim Gegenstand der Erfindung ausgegangen.

Jeder Katheterwechsel birgt das Risiko einer Verletzung der Harnröhre und das Einbringen von Keimen mit sich. Um Ersteres zu vermeiden, bestehen heutige transurethrale Blasenkatheter aus flexiblen Materialien wie Latex, PVC oder Silikon und sind zur Erhöhung der Gleitfähigkeit mit einer hydrophilen Beschichtung versehen. Auch werden heparinbeschichtete Blasenkatheter angeboten, die einer Inkrustation und bakteriellen Besiedlung des Katheters entgegenwirken und auf diese Weise Harnwegsinfektionen des Patienten weitgehend verhindern. In besonderen Fällen werden im klinischen Bereich zusätzlich Antibiotikagaben, z.B. durch ein freies Nebenlumen, verabreicht.

Ein neuartiger Lösungsansatz zum Hemmen oder Beseitigen der Entstehung einer Besiedlung durch Mikroorganismen, insbesondere Bakterien, in transurethralen Kathetern der vorliegenden Art ist aus der US 6,562,295 B1 entnehmbar. Hierbei sind auf der mit dem Körper in Kontakt kommenden äußeren zylindrischen Oberfläche des Blasenkatheters und der inneren Oberfläche des Hauptlumens photosensitive Substanzen (Photosensitizer) fest aufgebracht, die eine antimikrobielle Wirksamkeit entfalten, wenn sie mit Licht beaufschlagt werden. Eine lichtleitfähige Faser verbunden mit einem medizinischen Laser bestimmter Wellenlänge kann in einen oder mehreren Nebenlumen eingeführt und herausgezogen werden, um den Photosensitizer zu aktivieren und die sich auf den behafteten Oberflächen befindlichen oder diese berührenden Bakterien abzutöten. Dazu muss der Katheter selbst aus transparentem oder zumindest der Wellenlänge entsprechendem lichtdurchlässigem Material bestehen. Weiterhin ist nachteilig, dass nach Aufbringen bzw. der Immobilisierung des Photosensitizers auf den Oberflächen des Katheters keine Dosierungsmöglichkeit des Photosensitizers selbst mehr gegeben ist, ohne einen risikobehafteten Katheterwechsel vorzunehmen. Zudem muss der Katheter vor Gebrauch in einer lichtundurchlässigen Ummantelung aufbewahrt werden, um vorzeitiges Bleichen zu verhindern. Von einer Durchführung einer antibakteriellen photodynamische Behandlung (PdT) durch eine zeitlich und portionsmäßig wählbare Zugabe eines Photosensitizers in die Blase selbst und dessen dortige Aktivierung, wie es dem vorliegenden Erfindungsgegenstand zu eigen ist, ist keine Rede.

Die DE 601 12 434 T2 offenbart einen Katheter ähnlicher Bauart und gleicher Aufgabenstellung wie er aus der US 6,562,295 B1 bekannt ist, mit dem Unterschied, dass der Photosensitizer direkt in die Schlauchwand des Katheters eingebracht wurde und damit in ihr eingebettet ist. Eine außerhalb des Katheters angeordnete Lichtquelle und damit verkoppelte zahlreiche Lichtleitfasern zum Leiten von Licht entlang oder innerhalb der Katheterwand, um das erforderliche Licht zum Aktivieren des Photosensitizers bereitzustellen, werden vorgeschlagen. Da das Kathetermaterial auch hier lichtdurchlässig sein muss und der Photosensitizer ebenfalls fixiert ist und nur im Kontaktbereich mit dem Katheter seine Wirkung entfalten kann, bestehen die bereits oben, gegenüber dem aus der US 6,562,295 B1 bekannten Gegenstand genannten Nachteile ebenfalls. Die Durchführung einer photodynamischen Therapie (PdT) innerhalb der Blase selbst ist auch dieser Druckschrift fremd und kann daher den vorliegenden Erfindungsgegenstand ebenfalls nicht nahe legen.

Aus der US 2016/0213945 A1 sind Vorrichtungen bekannt, mit denen man nach Eingabe eines photoreaktiven Medikaments eine photodynamische Therapie (PdT) innerhalb der Blase durchführen kann. Diese in der Blase lichterzeugenden Vorrichtungen bestehen im Wesentlichen aus mindestens einem einlumigen flexiblen Katheter zum Einführen durch die Harnröhre bis in die Blase mit einer an ihrem proximalen, zum Körper hin liegenden Ende befindlichen Lichtquelle, z.B. einer oder mehrerer LEDs oder Laserdioden, und einer an ihrem distalen Ende mittels flexibler elektrischer Verbindung angeschlossenen steuernden Stromquelle.

In einem Ausführungsbeispiel der US 2016/0213945 A1 befindet sich eine der lichterzeugenden Vorrichtungen in einem üblichen 2-Wege-Katheter (Foley-Katheter) mit einem Hauptlumen zum Anschluss eines Urinbeutels und einem Nebenlumen zum Füllen eines Ballons zur Blockierung des in der Blase eingesetzten Katheters. Dabei ist eine LED, die über eine flexible Verbindungsleitung mit einer außerhalb des Katheters befindlichen, regelbaren Stromquelle verbunden ist, in den Nebenlumen bis zu einer proximalen Position einschiebbar, so dass die Prostata belichtet werden kann. Eine Benutzung dieser Vorrichtung innerhalb eines nach einer Prostataoperation verwendeten, herkömmlichen 3-Wege Katheter ist ebenfalls angedacht.

Keine der in der US 2016/0213945 A1 genannten Vorrichtungen offenbart eine gesteuerte Zufuhr portionierbarer Mengen eines Photosensitizers in die Blase selbst, um dort eine PdT durchzuführen, wie es wesentliches Merkmal der vorliegenden Erfindung ist. Zudem führt die US 2016/0213945 vom vorliegenden Erfindungsgegenstand weg, da mehrfach darauf hingewiesen wird, dass der verwendete Photosensitizer intravenös injiziert oder oral verabreicht wird.

WO2019/088940 A2 offenbart einen transurethralen Blasenatheter nach dem Oberbegriff des Anspruchs 1.

WO2019/088940 A2 offenbart einen Blasenkatheter für die Drainage und die photodynamische Therapie und andere photoinitiierte Behandlungen mit einem Haupt- und einem dazu parallel verlaufenden Nebenlumen und einem steuerbaren Leuchtmittel, wobei sich außerhalb des Blasenkatheters eine schalt- und/oder steuerbare Stromquelle zur Steuerung des Leuchtmittels befindet.

US 2018/369603 A1 offenbart einen ähnlichen Blasenkatheter zu WO2019/088940 A2 für die photodynamische Therapie, welcher ebenfalls ein Leuchtmittel umfasst, wobei die Stromkabel des Blasenkatheters in einem Verteiler mit einer Fluidleitung zu einer Leitung zusammengeführt werden. Diese Leitung wird in einem weiteren Verteiler mit weiteren Leitungen zum Katheter verbunden.

CN 201 135 707 Y offenbart ebenfalls einen Blasenkatheter für die Behandlung von Prostataerkrankungen und des Urethalsyndroma , welcher ein Leuchtmittel umfasst, wobei die elektrischen Kabel des Blasenkatheters in ein Nebenlumen geführt werden, das separat von einem, mit einem Dosierspender verbundenem, Lumen ist.

### Aufgabe und Lösung

Demgemäß ist es Aufgabe der vorliegenden Erfindung, die aus dem Stand der Technik bekannten, genannten Nachteile zu vermeiden und einen transurethralen Katheter zu schaffen, der sowohl das Abführen von Urin aus der Blase als auch eine zeitlich und portionsmäßig steuerbare photodynamische Therapie zur Abtötung bakterieller Keime innerhalb der Blase ermöglicht.

Diese Aufgabe wird durch einen transurethralen Blasenkatheter nach dem vorliegenden Patentanspruch 1 gelöst. Vorteilhafte Ausgestaltungen und Weiterbildungen des mit dem Patentanspruch 1 beanspruchten transurethralen Blasenkatheters sind Gegenstand der Unteransprüche 2 bis 10.

### Vorteile der Erfindung

Neben der Lösung der zugrunde liegenden Aufgabe zeichnet sich der transurethrale Blasenkatheter nach dem vorliegenden Patentanspruch 1 dadurch aus, dass er in seinem Aufbau von herkömmlichen, bereits vielfach bekannten und bewährten, mit einem freien Lumen versehenen Blasenkathetern (z.B. Foleykathetern) ausgeht und diese in vorteilhafter Weise als Basiselement nutzen kann. Darüber hinaus ergibt sich bei der medizinischen Auswertung eines Röntgenbildes auf Grund der höheren Dichte der in das Nebenlumen eingesteckten metallischen Bestandteile des Stromkabels und der Leuchtmittelfassung eine bessere Erkennbarkeit der Lage des Blasenkatheters im Körper des Patienten.

Die im Patentanspruch 2 angegebenen Merkmale ermöglichen neben Durchführungen von photodynamischen Therapien (PdT) auch Reinigungsvorgänge in Katheter und Blase oder weitere Therapien durch Gaben spezieller Medikamente. So werden insbesondere nach transurethralen Operationen und/oder bei starken Blutungen in der Harnblase Spüllungen benutzt, um einer Gerinnung des Blutes vorzubeugen, damit Katheterverschlüsse durch Inkrustation des Katheters oder Ablagerungen in der Blase möglichst vermieden werden, was zu folgenschweren Unterbrechungen der Ableitung des Urinabflusses führen könnte. Diese Spülungen sind ohne einen, mit der zusätzlichen Gefahr des Eindringens von Keimen verbundenen Katheterwechsel durchführbar. Erfahrungsgemäß steigt die Gefahr einer bakteriellen Besiedlung des Blasenkatheters, der Harnwege oder der Blase selbst je länger ein Katheter liegt. Hierbei werden in Spezialfällen diese Infektionen oftmals mit Antibiotika behandelt. Es ist daher ein weiterer Vorteil, dass derartiger Therapien auch mit dem Gegenstand des vorliegenden Patentanspruch 2 durchführbar sind.

In vorteilhafter Weiterbildung der Erfindung können mit den im Patentanspruch 3 genannten Merkmalen portions- und lichtmäßig, manuell steuerbare photodynamische Behandlungen durchgeführt werden oder im Bedarfsfall portionierte Mengen, der in den Reservoirs jeweils befindlichen speziellen Flüssigkeiten, in den Katheter ausgegeben werden. Sind mehrere, mit unterschiedlichen Flüssigkeiten gefüllte Reservoire im Dosierspender angeordnet (Multi-Reservoir-Dosierspender), kann der behandelnde Arzt wahlweise eine entsprechende Therapie oder eine Spülung durchführen. Bei nur einem im Dosierspender angeordneten Reservoir (Single-Reservoir-Dosierspender) ergibt sich aufgrund der enthaltenen größeren Flüssigkeitsmenge eine erweiterte freie Dosierbarkeit zur manuell gesteuerten patientenbezogenen Ausgabe.

Die im Patentanspruch 4 angegebenen Merkmale offenbaren eine automatisch den Dosierspender und das Leuchtmittel steuernde, Mikrocontroller beinhaltende Aktorik zur Durchführung der im Patentanspruch 3 genannten, manuell steuerbaren Behandlungen. Die hiermit vorliegende zweckmäßige Ausgestaltung des Gegenstandes nach den Patentansprüchen 1 oder 2 weist auch weiterhin alle bereits genannten Vorteile des Gegenstandes des vorliegenden Patentanspruchs 3 auf.

Der Gegenstand des vorliegenden Patentanspruchs 5 schafft die Möglichkeit auch konventionelle, manuell handhabbare und mit einer Skala zur Bestimmung der in ihnen enthaltenen Flüssigkeitsmenge versehene Spritzen als automatisierte Dosierspender beim Erfindungsgegenstand zur Durchführung einer photodynamischen Therapie einzusetzen. Größere Inhaltsmengen und freiere Dosierbarkeit sind weitere Vorteile dieser Alternative.

Patentanspruch 6 zeigt eine konstruktive Ausgestaltung der Stromquelle zur Steuerung des Leuchtmittels für Gegenstände nach den vorliegenden Patentansprüchen 3 oder 5, die trotz ihres einfachen Aufbaus eine vorteilbringende Intervallsteuerung des Leuchtmittels und eine Statusanzeige beinhaltet.

Die Merkmale des Patentanspruchs 7 beugen einer Verwechslungsgefahr der Reservoirinhalte vor und lassen die noch vorhandene Flüssigkeitsmenge erkennen.

Das Merkmal des Patentanspruchs 8 sorgt für einen flüssigkeitsdichten Verschluss und ermöglicht zugleich die Benutzung von Blasenkathetern mit Nebenlumen unterschiedlicher Durchmesser.

Patentanspruch 9 nennt als Leuchtmittel eine LED mit spezieller Charakteristik und zeigt deren spezielle Halterung am Stromkabel auf, wodurch sich eine vorteilhafte Beleuchtung in der Blase und eine flüssigkeitsdichte, sichere Befestigung ergeben.

Durch die Anzahl der im Patentanspruch 10 genannten, bereits auf dem Markt befindlichen, verwendbaren Photosensitizern ist eine vorteilhafte Auswahlmöglichkeit gegeben; das weitere im Patentanspruch 10 aufgeführte Merkmal einer Anpassung der Wellenlänge des Leuchtmittels an den verwendeten Photosensitizer verbessert die Aktivierung desselben.

### Ausführungsbeispiele

Im Folgenden werden bevorzugte Ausführungsformen der Erfindung unter Bezugnahme auf Figuren 1 bis 8 näher erläutert.

Es zeigen:
- Fig. 1:: Einen Herkömmlichen 3-Wege-Blasenkatheter mit angeschlossenem Urinbeutel
- Fig. 2:: Einen Transurethralen Blasenkatheter nach der Erfindung mit Single-Reservoir Dosierspender und manueller Lichtsteuerung
- Fig. 3:: Ein LED-Leuchtmittel
- Fig. 4:: Einen Blasenkatheter mit Dosierspender in Form eines Multi-Reservoirs und manueller Lichtsteuerung
- Fig. 5:: Einen Blasenkatheter mit automatischer Licht- und Dosierspender-Steuerung für ein Multi-Reservoir oder ein Single-Reservoir
- Fig. 6:: Eine Aktorikeinheit mit Schaltschema für Automatikbetrieb
- Fig. 7:: Zeitliche Schaltabläufe der Gaben von Photosensitizern
- Fig. 8:: Einen Blasenkatheter mit Infusionsspritzenpumpe, manuell oder automatisch steuerbar

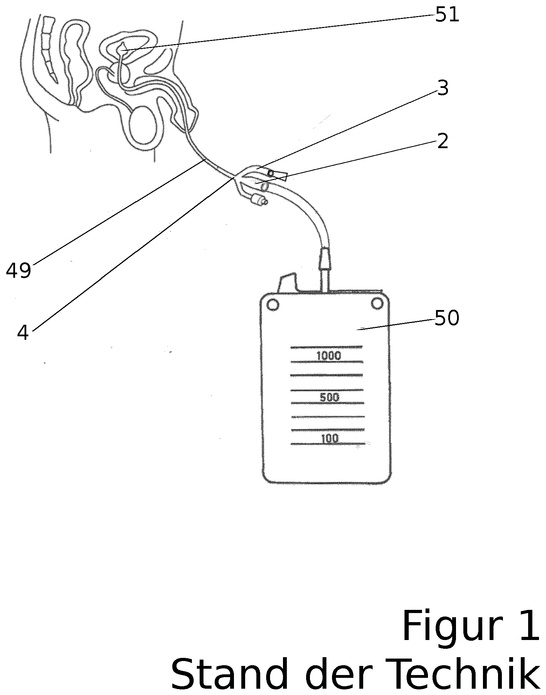

Figur 1 zeigt einen im Stand der Technik verwendeten transurethralen 3-Wege-Katheter (49). Dieser besitzt ein Hauptlumen (2) zur Harnableitung und mindestens ein freies, dazu parallel verlaufendes Nebenlumen (3). An das Hauptlumen (2) ist ein Urinbeutel (50) mittels seines Urinschlauches in bekannter Weise angeschlossen. Weiterhin besitzt der 3-Wege-Katheter (49) an seinem proximalen Ende einen aufblasbaren Ballon (51). Dieser ist, z. B. mittels einer nicht dargestellten Spritze, die am distalen Ende eines zweiten Nebenlumens anschließbar ist, mit sterilem, destillierten Wasser füllbar, wodurch ein unerwünschtes Herausrutschen des 3-Wege-Katheters (49) aus der Blase blockiert wird.

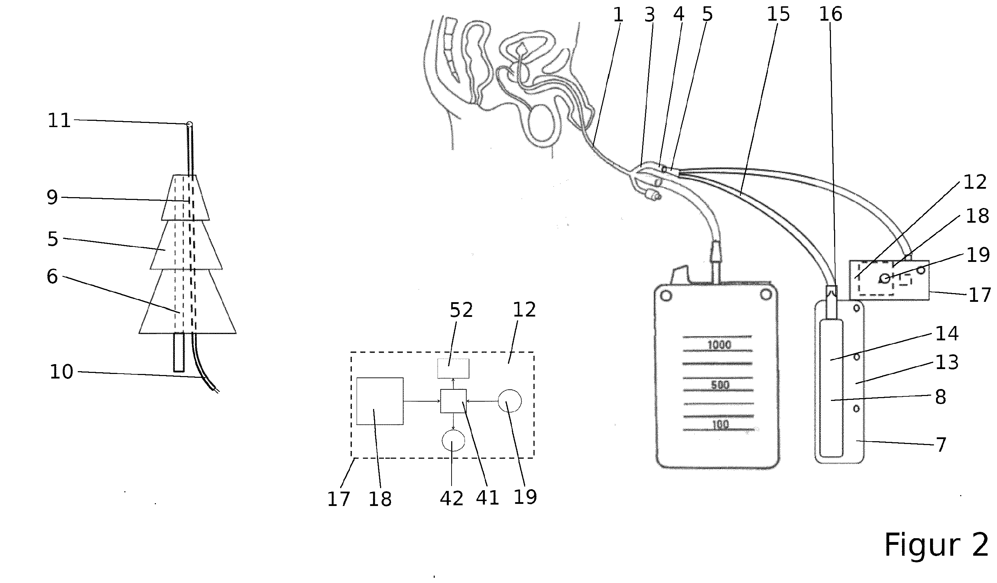

Figur 2 zeigt einen erfindungsgemäßen transurethralen Blasenkatheter (1) mit einem manuell betätigbaren Dosierspender (7) und manueller Lichtsteuerung eines Leuchtmittels (11), wobei ein Stopfen (5) sowie eine Stromquelle (12) als Einzelheiten herausgehoben, vergrößert dargestellt sind.

Der transurethraler Blasenkatheter (1) besitzt am distalen Ende (4) des freien Nebenlumens (3) einen einsteckbaren, nach außen flüssigkeitsdichten Stopfen (5), der einen Durchgangskanal (6) mit einem damit fest verbundenen Dosierspender (7) für einen Photosensitizer (8) aufweist, wobei der Dosierspender (7) bei Bedarf zusätzliche weitere Flüssigkeiten wie Spüllösungen oder spezielle Medikamente, z.B. Antibiotika, enthalten kann.

Der Dosierspender (7) besteht aus einer lichtundurchlässigen blisterartigen Verpackung (13), die im Inneren ein manuell ausdrückbares Reservoir (14), welches mit einer zum Durchgangskanal (6) führenden Ausgabeleitung (15) verbunden ist und an ihrer ausgangsseitigen Verbindung ein Schnabelventile (16) mit einer Öffnungsrichtung zur Ausgabeleitung (15) hin besitzt und zur Aufbewahrung und Ausgabe portionierter Mengen von im Dosierspender (7) jeweils befindlichen Flüssigkeiten dient.

Der Stopfen (5) verfügt weiterhin auch über eine flüssigkeitsdichte, form- und/oder materialschlüssige Durchführung (9) für ein zweiadriges, biokompatibles Stromkabel (10), wobei dessen in das Nebenlumen (3) eingesteckter Teil einschließlich einem damit an seinem Ende verbundenen Leuchtmittel (11) die Länge des Nebenlumens (3) aufweist. Am anderen, außerhalb des Blasenkatheters (1) befindlichen Ende des Stromkabels (10) befindet sich eine schalt- und/oder steuerbare Stromquelle (12) zur Steuerung des Leuchtmittels (11). Die Stromquelle (12) befindet sich in einem luft- und flüssigkeitsdichten sowie Desinfektionsmittel resistenten Gehäuse (17) und hat eine Stromversorgung (18) mit zugehörigem, von außen betätigbaren Bedienelement (19). Das Gehäuse (17) beinhaltet weiterhin einen Mikrocontroller (41) und verfügt über eine Kontrolllampe (42) und eine Anschlussbuchse (52) für das Stromkabel (10).

Nachdem der Blasenkatheter (1) in den Harnleiter des Patienten eingeführt, der Ballon (51) aufgebläht und der Urinbeutel (50) angesteckt wurde, kann Stopfen (5) mit seinem Leuchtmittel (11) in das freie Nebenlumen (3) eingeführt werden. Aufgrund der Länge des Stromkabels (10) gelangt das Leuchtmittel (11) bis an das proximale Ende des Blasenkatheters (1).

Am Stopfen (5) ist der Dosierspender (7) angeschlossen. Der Durchmesser des Nebenlumens (3) ist so bemessen, dass durch dieses gleichzeitig das Stromkabel (10) geführt wird und Flüssigkeit aus dem Dosierspender (7) in die Blase fließen kann.

Die behandelnde Person kann manuell auf das Reservoir (14) drücken, wodurch Photosensitizer (8) durch das Schnabelventil (16), die Ausgabeleitung (15), das Nebenlumen (3) in die Blase fließt. Während dieses Vorganges oder danach betätigt die behandelnde Person das Bedienelement (19), wodurch das Leuchtmittel (11) zum Leuchten gebracht wird. Die Zeitdauer dieses Leuchtens kann durch weiteres Schalten am Bedienelement (19) beendet werden. Der Mikrocontroller (41) kann alternativ die Zeitdauer des Leuchtens des Leuchtmittels (11) steuern, wobei diese Zeitdauer mittels dem Bedienelement (19) einstellbar ist. Leuchtet die Kontrolllampe (42) grün, dann ist das Gerät in Betrieb und in Ordnung, blinkt sie grün, leuchtet das Leuchtmittels (11) und leuchtet die Kontrolllampe (42) rot, liegt eine Störung vor. Leuchtet die Kontrolllampe (42) nicht, ist die Stromquelle (12) ausgeschaltet.

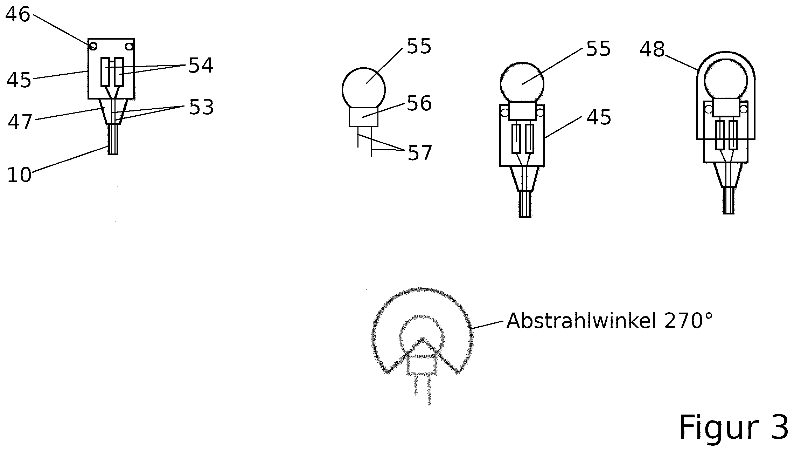

Figur 3 zeigt in mehreren Darstellungen das aus einer LED bestehende Leuchtmittel (11). In der linken Darstellung ist das Stromkabel (10) zu sehen. Es mündet in einen Stecker (45) mit O-Ring Dichtung (46), wobei der Stecker (45) an der Eintrittsstelle des Stromkabels (10) einen Flüssigkeit abdichtenden Knickschutz (47) am ummantelten Stromkabel (10) besitzt. Die Adern (53) des Stromkabels (10) enden an Kontakten (54). In der Darstellung rechts daneben ist der LED-Leuchtkörper (55) mit seinem Sockel (56) und seinen Anschlussfüßen (57) zu sehen. Daneben ist der auf den Stecker aufgesteckte LED-Leuchtkörper (55) zu sehen. Darauf ist, wie weiter rechts daneben dargestellt, eine lichtdurchlässige Kuppel (48) aufgesteckt oder aufgeschraubt. Die Darstellung darunter deutet an, dass der LED-Leuchtkörper (55) einen allseitigen Abstrahlwinkel von 270° besitzt und so einen weiten Bereich in der Blase ausleuchtet bzw. den Photosensitizer (8) aktiviert. Die vom LED-Leuchtkörper (55) abgestrahlte Frequenz ist auf den Photosensitizer (8) so abgestimmt, dass dieser seine optimale Wirkung entfaltet.

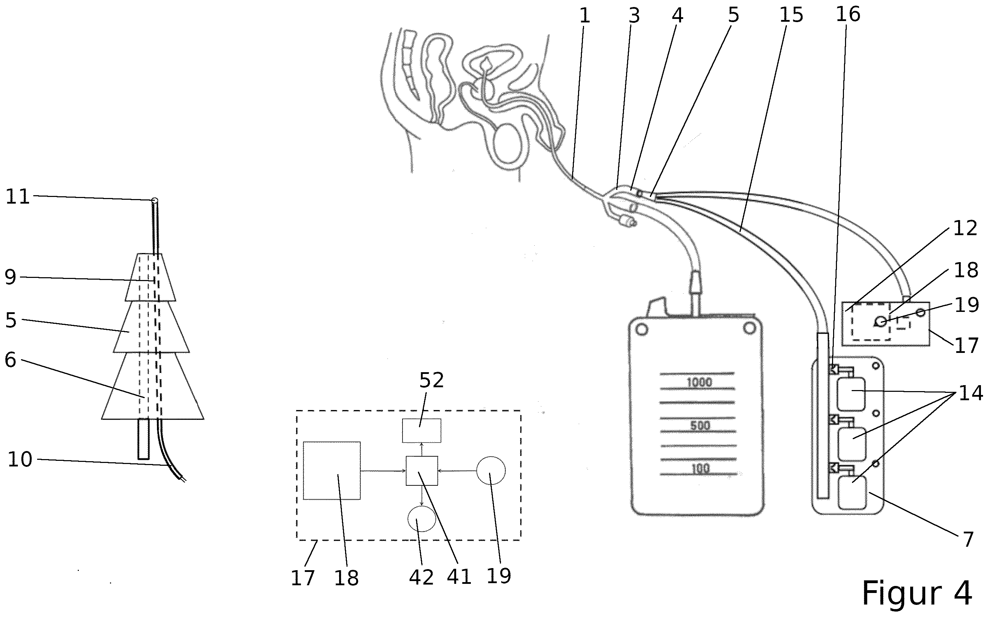

Figur 4 zeigt einen Blasenkatheter nach der Erfindung mit einem Dosierspender in Form eines Multi-Reservoir-Dosierspenders und manueller Lichtsteuerung. Bis auf den Dosierspender (7) entspricht diese Ausführungsform der in Figur 2 beschriebenen, sodass hier nur der unterschiedliche Dosierspender (7) zu beschreiben ist. Auch hier besteht der Dosierspender (7) aus einer lichtundurchlässigen blisterartigen Verpackung (13), die im Inneren mehrere, vorzugsweise drei, manuell ausdrückbare Reservoire (14), aufweist, die jeweils an ihren ausgangsseitigen Verbindungen Schnabelventile (16) mit einer Öffnungsrichtung zur Ausgabeleitung (15) besitzen. Die Reservoire (14) dienen zur Aufbewahrung und Ausgabe portionierter Mengen (Bolusgaben) der sie jeweils beinhaltenden Flüssigkeit. Die Reservoire (14) können farblich unterschiedliche Kennzeichnungen entsprechend ihres Inhalts aufweisen und mit einer Skala zum Erkennen der in ihnen befindlichen Flüssigkeitsmenge versehen sein. Dies erleichtert das Erkennen der unterschiedlichen Flüssigkeiten in den Reservoiren (14). In den verschiedenen Reservoiren (14) können als Photosensitizer (8) Dyes, wie Acridine orange, Methyline blue, Rose Bengal, Toludine blue,oder Porphyrine, wie Photofrin, Fotoscan, Aminolevulinic acid, Temoporfin, Talaporfin sodium, oder Chlorine, wie Chlorin e6, oder Furocumarine, wie Psoralen, oder Xanthene, wie Erythrosine, oder Monopene, wie Azulene, eingesetzt werden.

Die Verwendung dieser Ausführungsform erfolgt grundsätzlich so, wie zu Figur 2 beschrieben. Der Unterschied besteht allerdings darin, dass die Abgabe von drei unterschiedlichen Flüssigkeiten in wählbarer Reihenfolge möglich ist, ohne das Nebenlumen (3) des Blasenkatheters (1) zu öffnen.

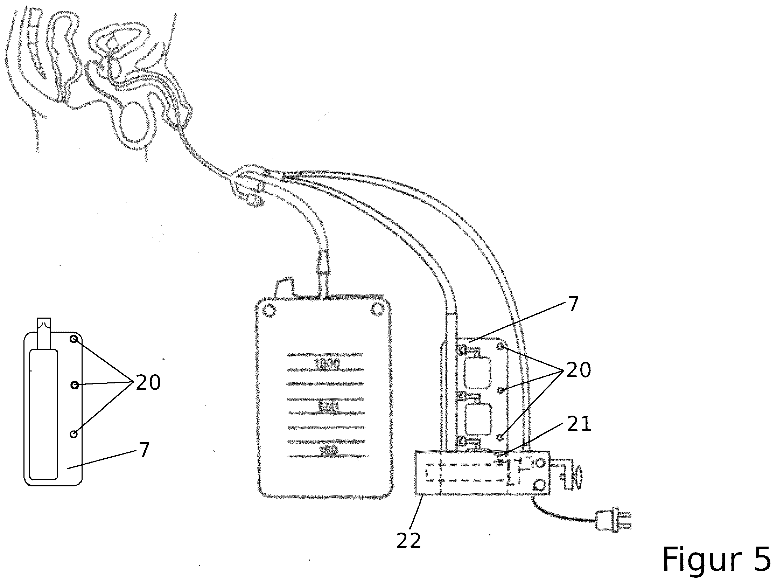

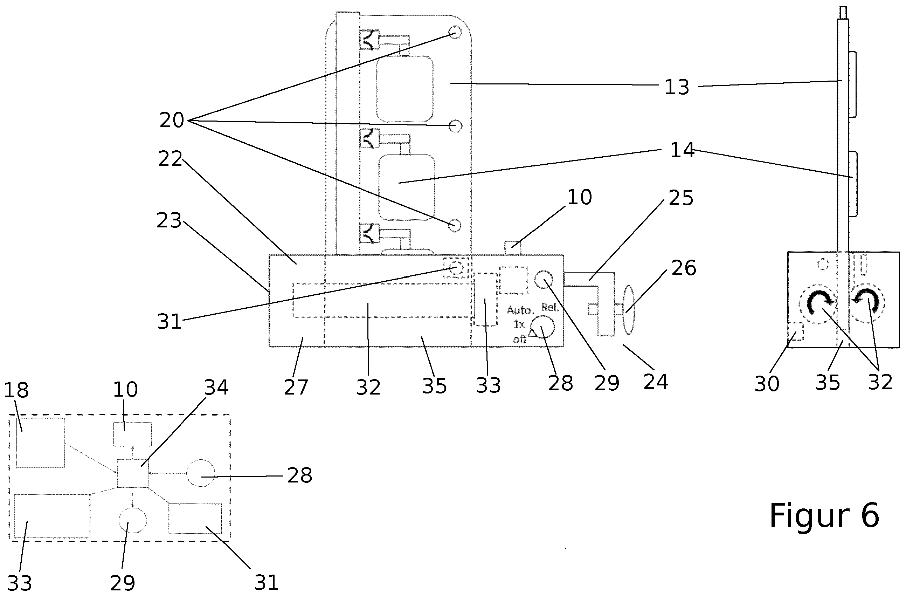

Figur 5 zeigt einen Blasenkatheter nach der Erfindung mit automatischer Licht- und Dosierspender-Steuerung für Multi-Reservoire oder Single-Reservoire. Ausgehend von der Beschreibung der Figur 2 werden ausschließlich die dargestellten Unterschiede beschrieben. So hat der Dosierspender (7) drei kreisförmige Durchbrüche (20), die in konstantem Abstand vorhanden sind. Eine in Figur 6 näher beschriebene Aktorikeinheit (22) verfügt über eine Lichtschranke (21). Sie gibt ein Signal ab, wenn ihr Lichtstrahl mittels der Durchbrüche (20) nicht unterbrochen ist. Dadurch ist die jeweilige Position des Dosierspenders (7) jederzeit nach einer Bolusgabe bestimmbar. Dies gilt für beide Ausführungen des Dosierspenders (7), als Multi-Reservoir (14) oder als Single-Reservoir (14).

Figur 6 zeigt die Aktorikeinheit (22) mit dem Schaltschema für Automatikbetrieb. Die Aktorikeinheit (22) umfasst ein quaderförmiges Gehäuse (23). Dieses hat an der einen Stirnseite eine fest angefügte Schraubklemme (24), bestehend aus einem Winkel (25) und einer Feststellschraube (26), zur Befestigung des Gehäuses (23) an einem geeigneten Ort, wie beispielsweise Bettrahmen oder Infusionsständer. Die Frontfläche (27) des Gehäuses (23) besitzt einen in 4 Stellungen drehbaren Bedienschalter (28). Dieser unterbricht die Stromzufuhr in der Stellung "off", wodurch keine Aktivitäten möglich sind. In der Stellung "1x" wird einmal Photosensitizer (8) abgegeben und das Leuchtmittel (11) für einen definierten Zeitraum aktiviert. In der Stellung "Auto" des Bedienschalters (28) wird in definierten Zeitintervallen immer wieder eine Behandlung durchgeführt. Die Stellung "Rel." bewirkt eine Umkehr der Bewegungsrichtung der Verpackung (13), sodass diese in der Darstellung nach oben herausbewegt werden kann, um eine neue Verpackung (13) wieder einzuführen. Neben dem Bedienschalter (28) befindet sich eine Kontrolllampe (29). Diese ist stromlos wenn, die Aktorikeinheit (22) ausgeschaltet ist. Die Kontrolllampe (29) leuchtet grün auf, wenn die Aktorikeinheit (22) in Betrieb ist und alle Funktionen in Ordnung sind. Ein blinkendes Grün signalisiert den Automatikmodus der Aktorikeinheit (22). Eine rot leuchtende Kontrolllampe (29) zeigt eine Störung der Aktorikeinheit (22) an.

In dem Gehäuse (23) sind in seiner Längsrichtung zwei parallel ausgerichtete, beabstandete Walzen (32) mit elektromotorischem Antrieb (33) eingebaut. Der Walzenabstand ist so bemessen, dass die drehenden Walzen (32) die zwischen ihnen befindliche Verpackung (13) in zwei Richtungen bewegen können. In der gezeigten Darstellung wird die Verpackung (13) nach unten gezogen und dabei der Photosensitizer (8) aus den Reservoiren (14) herausgepresst. Sind die Reservoire (14) geleert, wechselt die Drehrichtung der Walzen (32) und die Verpackung (13) ist entnehmbar. Damit dieser Vorgang möglich ist, verfügt das Gehäuse (23) über einen schlitzartigen, rechteckigen Durchbruch (35) zum Durchfahren der Verpackung (13).

Im Gehäuse (23) ist mindestens eine Lichtschranke (31) zum Erfassen bzw. Scannen der in der Verpackung (13) vorhandenen Durchbrüche (20) positioniert. Die Oberseite des Gehäuses (23) verfügt über eine Durchführung des Stromkabels (10) zum Anschluss des Leuchtmittels (11).

An der der Frontfläche (27) gegenüberliegenden Rückseite ist ein mit einem Stecker ausgestatteter Stromversorgungsanschluss (30) angebracht.

Im Gehäuse (23) ist zur Ausgabe von Bolusgaben aus dem Dosierspender (7) ein diesen steuernder Mikrocontroller (34) mit einer Stromversorgung (18) untergebracht. Der Mikrocontroller (34) wird mittels Bedienschalter (28) aktiviert und steuert über den Anschluss des Stromkabels (10) den elektromotorischen Antrieb (33) der Walzen (32) sowie die Leuchtdauer des Leuchtmittels (11) für definierte Zeitintervalle. Dabei nutzt er die von der Lichtschranke (31) kommenden Signale als zusätzliche Eingangssteuersignale.

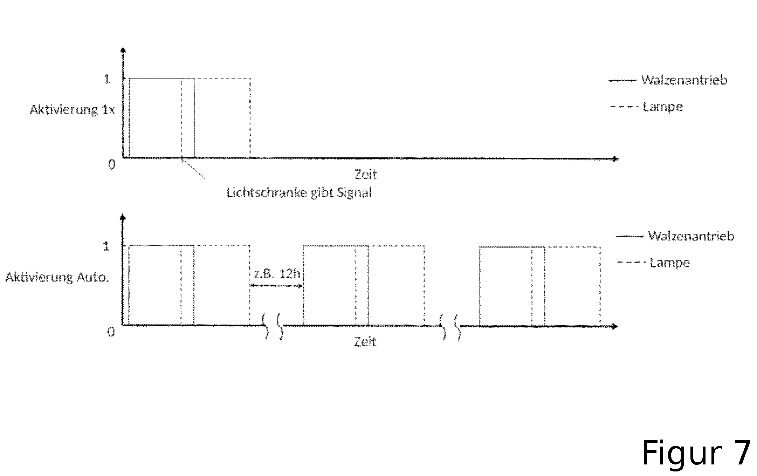

Figur 7 zeigt beispielhaft zeitliche Schaltabläufe der Gabe von Photosensitizern (8). Beim oben dargestellten Vorgehen nach der Ausgestaltung gemäß Figur 6 werden, wenn der Bedienschalter (28) in der Stellung "1x" ist, die Walzen (32) zeitlich begrenzt angetrieben, wobei die Verpackung (13) von den Walzen (32) eingezogen wird. Gegen Ende dieses Vorganges gelangt ein Durchbruch (20) in die Lichtschranke (31), wodurch das Leuchtmittel (11) für ein Zeitintervall eingeschaltet wird. Kurz danach endet das Zeitintervall für den Betrieb der Walzen (32). Nach Ablauf des Zeitintervalls für das Leuchtmittel (11) wird dieses abgeschaltet. Die entsprechende Steuerung ist in der Stromquelle (12) vorhanden. Dieser Ablauf kann nach Belieben wiederholt werden. In Stellung "Auto" des Bedienschalters (28) erfolgt der vorstehend beschriebene Ablauf in gleicher Weise, nur das dieser automatisch wiederholt wird. Das Zeitintervall für die Wiederholung kann 12 Stunden betragen. Grundsätzlich sind die Längen der Zeitintervalle individuell einstellbar.

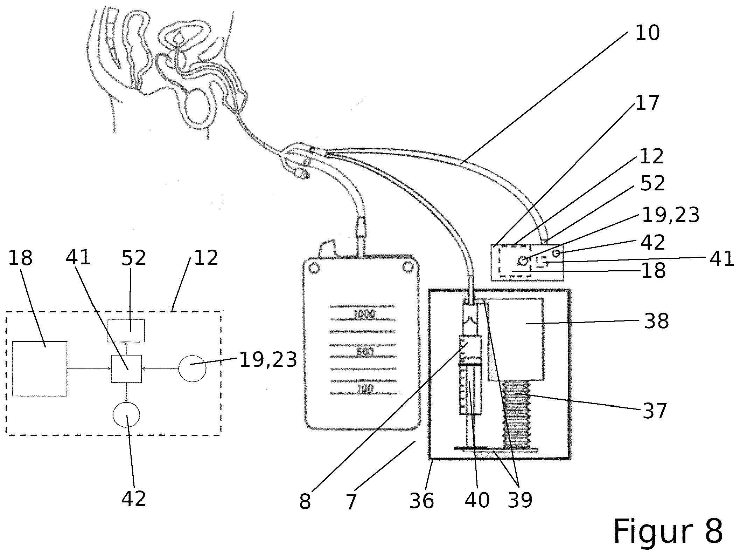

Figur 8 zeigt einen Blasenkatheter mit Infusionsspritzenpumpe, die manuell oder automatisch steuerbar ist. Auch bei dieser Ausführungsform ist das Stromkabel (10) mit einer Stromquelle (12) verbunden. Auch diese besteht aus einem luft- und flüssigkeitsdichten sowie Desinfektionsmittel resistenten Gehäuse (17). In ihm befindet sich eine Stromversorgung (18) mit zugehörigem, von außen betätigbaren Bedienelement (19). Das Gehäuse (17) besitzt die gleichen Merkmale, wie die in Figur 2 beschriebenen Gehäusemerkmale.

Der Dosierspender (7) besteht aus einem Gehäuse (36), in welchem ein motorgetriebener Spindeltrieb (37) mit Winkelschrittgeber (38) und mit axial gegenüberliegenden Endflanschen (39) eingebaut ist. In der Endstellung der Endflansche (39) ist zwischen ihnen eine vollgefüllte Spritze (40) mit einem Photosensitizer (8) befestigbar. Wie üblich ist diese Spritze (40) mit einer Skala ausgestattet, um die eingespritzte Flüssigkeitsmenge anzuzeigen.

Mittels eines nicht dargestellten Schalters wird der Spindeltrieb (37) eingeschaltet, bis die gewünschte Bolusgabe erfolgt ist. Danach wird er wieder ausgeschaltet.

In der Stromquelle (12) ist ein Mikrocontroller (41) und eine Stromversorgung (18) untergebracht, die ein handelsüblicher Akku sein kann. Auch weist die Stromquelle (12) ein Bedienelement (19) auf, das als On/Off-Schalter (43) ausgeführt ist, sowie eine Kontrolllampe (42). Mittels einer Anschlussbuchse (52) ist das Leuchtmittel (11) angeschlossen.

Der On/Off-Schalter (43) startet oder unterbricht die Stromzufuhr zum Mikrocontroller (41) und zum Leuchtmittel (11).

Im Falle eines automatischen Betriebes hat der Mikrocontroller (41) zusätzlich eine Verbindung mit dem Spindeltrieb (37) und dem Winkelschrittgeber (38). Über den Mikrocontroller (41) sind die Laufzeiten des Spindeltriebes (37) schaltbar, wobei Signale des Winkelschrittgebers (38) den Verfahrweg des Endflansches (39) angeben, welches ein Maß für die Menge der Bolusgabe ist. Die Stromversorgung des Leuchtmittels (11) erfolgt wie in der bei Figur 7 beschriebenen Weise.

### Bezugszeichenliste

- 1: Blasenkatheter
- 2: Hauptlumen
- 3: Nebenlumen
- 4: distales Ende
- 5: Stopfen
- 6: Durchgangskanal
- 7: Dosierspender
- 8: Photosensitizer
- 9: Durchführung
- 10: Stromkabel
- 11: Leuchtmittel
- 12: Stromquelle
- 13: Verpackung
- 14: Reservoire
- 15: Ausgabeleitung
- 16: Schnabelventile
- 17: Gehäuse (für Stromquelle)
- 18: Stromversorgung
- 19: Bedienelemente
- 20: Durchbrüche
- 21: Lichtschranke
- 22: Aktorikeinheit
- 23: Gehäuse (für Aktorikeinheit)
- 24: Schraubklemme
- 25: Winkel
- 26: Schraubklemme
- 27: Frontfläche
- 28: Bedienschalter
- 29: Kontrolllampe
- 30: Stromversorgungsanschluss
- 31: Lichtschranke
- 32: Walzen
- 33: elektromotorischer Antrieb
- 34: Mikrocontroller
- 35: schlitzartiger, rechteckiger Durchbruch
- 36: Gehäuse (für Dosierspender)
- 37: motorgetriebener Spindeltrieb
- 38: Winkelschrittgeber
- 39: Endflansche
- 40: Spritze
- 41: Mikrocontroller
- 42: Kontrolllampe
- 43: On/Off-Schalter
- 44: Stufentülle
- 45: Stecker
- 46: Dichtung
- 47: Knickschutz
- 48: Kuppel
- 49: 3-Wege-Katheter
- 50: Urinbeutel
- 51: Ballon
- 52: Anschlussbuchse
- 53: Adern
- 54: Kontakte
- 55: LED-Leuchtkörper
- 56: Sockel
- 57: Anschlussfüße

## Patentansprüche

1. Transurethraler Blasenkatheter (1) mit einem Hauptlumen (2) zur Harnableitung und mindestens einem freien, dazu parallel verlaufenden Nebenlumen (3), und mit einem zweiadrigen, biokompatiblen Stromkabel (10), wobei sich am außerhalb des Blasenkatheters (1) befindlichen Ende des Stromkabels (10) eine schalt- und/oder steuerbare Stromquelle (12) zur Steuerung eines Leuchtmittels (11) befindet, **dadurch gekennzeichnet, dass**
- das freie Nebenlumen (3) am distalen Ende (4) des Blasenkatheters (1) einen einsteckbaren flüssigkeitsdichten Stopfen (5) besitzt, der sowohl einen Durchgangskanal (6) mit einem damit fest verbundenen Dosierspender (7) für einen Photosensitizer (8) und bei Bedarf für weitere, zusätzliche Flüssigkeiten als auch eine flüssigkeitsdichte, form- und/oder materialschlüssige Durchführung (9) für das zweiadrige. biokompatible Stromkabel (10) hat, wobei dessen in das Nebenlumen (3) eingesteckter Teil einschließlich dem damit am Ende verbundenen Leuchtmittel (11) die Länge des Nebenlumens (3) aufweist.

2. Transurethraler Blasenkatheter (1) nach Patentanspruch 1,
**dadurch gekennzeichnet, dass**
- der Dosierspender (7) neben Photosensitizern (8) zusätzlich vorhandene Flüssigkeiten wie Spüllösungen oder spezielle Medikamente für verschiedene Therapien enthält.

3. Transurethraler Blasenkatheter (1) nach einem der Patentansprüche 1 oder 2,
**dadurch gekennzeichnet, dass**
- der Dosierspender (7) aus einer lichtundurchlässigen blisterartigen Verpackung (13) besteht, die im Inneren ein oder mehrere angeordnete, manuell ausdrückbare Reservoire (14), welche mit einer zum Durchgangskanal (6) führenden Ausgabeleitung (15) verbunden sind, jeweils an ihren ausgangsseitigen Verbindungen Schnabelventile (16) mit einer Öffnungsrichtung zur Ausgabeleitung (15) besitzen und zur Aufbewahrung und Ausgabe portionierter Mengen der sie jeweils beinhaltenden Flüssigkeit dienen, aufweist,
- und
- sich die mit dem Stromkabel (10) verbundene Stromquelle (12) in eine luft- und flüssigkeitsdichten sowie Desinfektionsmittel resistenten Gehäuse (17) befindet und aus einer Stromversorgung (18) mit zugehörigen, von außen betätigbaren Bedienelementen (19) besteht.

4. Transurethraler Blasenkatheter (1) nach einem der Patentansprüche 1 oder 2,
**dadurch gekennzeichnet, dass**
- der Dosierspender (7) aus einer lichtundurchlässigen blisterartigen Verpackung (13) besteht, die im Inneren ein oder mehrere angeordnete, maschinell ausdrückbare Reservoire (14), welche mit einer zum Durchgangskanal (6) führenden Ausgabeleitung (15) verbunden sind, jeweils an ihren ausgangsseitigen Verbindungen Schnabelventile (16) mit einer Öffnungsrichtung zur Ausgabeleitung (15) besitzen und zur Aufbewahrung und Ausgabe portionierter Mengen der sie jeweils beinhaltenden Flüssigkeit dienen, aufweist,
- die Verpackung (13) selbst Durchbrüche (20) als Hilfsmarkierungen für eine zur Bewegungssteuerung des Dosierspenders (7) benötigte Lichtschranke (21 ) enthält,
und
- eine automatisch, den Dosierspender (7) und das Leuchtmittel (11) steuernde Aktorikeinheit (22) in einem quaderförmigen Gehäuse (23) implementiert ist, dessen eine Stirnseite eine Schraubklemme (24), bestehend aus einem Winkel (25) und einer Feststellschraube (26), zur Befestigung des Gehäuses (23) besitzt, die Frontfläche (27) ein in 4 Stellungen drehbaren Bedienschalter (28) und eine Kontrolllampe (29) aufweist, die Oberseite über eine Durchführung des Stromkabels (10) zum Anschluss des Leuchtmittels (11) verfügt, an der der Frontfläche (27) gegenüberliegenden Rückseite ein mit einem Stecker ausgestatteter Stromversorgungsanschluss (30) angebracht ist, im Gehäuse (23) mindestens eine Lichtschranke (31) zum Erfassen der im Dosierspender (7) vorhandenen Durchbrüche (20), zwei in Längsrichtung des Gehäuses (23) parallel ausgerichtete, beabstandete Walzen (32) mit elektromotorischem Antrieb (33) zur Ausgabe von Bolusgaben aus dem Dosierspender (7) sowie einem diese steuernden Mikrocontroller (34), der gleichzeitig die Kontrolllampe (29) ansteuert und die Schaltzeiten des Leuchtmittels (11) bestimmt, untergebracht sind
und
- das Gehäuse (23) über einen schlitzartigen, rechteckigen Durchbruch (35) verfügt, durch den der zwischen den beiden Walzen (32) befindliche Dosierspender (7) in zwei Richtungen ziehbar ist.

5. Transurethraler Blasenkatheter (1) nach Patentanspruch 1,
**dadurch gekennzeichnet, dass**
- sich die mit dem Stromkabel (10) verbundene Stromquelle (12) in einer luft- und flüssigkeitsdichten sowie Desinfektionsmittel resistenten Gehäuse (17) befindet und aus einer Stromversorgung (18) mit zugehörigen, von außen betätigbaren Bedienelementen (19) besteht.
und
- der Dosierspender (7) aus einem Gehäuse (36) besteht, in welchem sich ein motorgetriebener Spindeltrieb (37) mit Winkelschrittgeber (38) und mit axial gegenüberliegenden Endflanschen (39) befindet, in deren Endstellung zwischen ihnen eine vollgefüllte Spritze (40) mit einem Photosensitizer (8) befestigbar ist

6. Transurethraler Blasenkatheter (1) nach Patentanspruch 3 oder 5,
**dadurch gekennzeichnet, dass**
- die Stromquelle (12) einen Mikrocontroller (41) zur Steuerung der Leuchtdauer des Leuchtmittels (11) über ein definiertes Zeitintervall besitzt und dieser gleichzeitig eine den Status der Stromquelle (12) anzeigende Kontrolllampe (42) ansteuert, als Stromversorgung (18) ein handelsüblicher Akku dient und als Bedienelement (19) ein On/Off-Schalter (43) vorhanden ist, der die Aktivierung des Leuchtmittels (11) über den Mikrocontroller (41) startet oder die Stromzufuhr zum Mikrocontroller (41) zum Ausschalten des LED-Leuchtmittels (11) unterbricht.

7. Transurethraler Blasenkatheter (1) nach einem der Patentansprüche 3 oder 4,
**dadurch gekennzeichnet, dass**
- die Reservoire (14) farblich unterschiedliche Kennzeichnungen entsprechend ihres Inhalts aufweisen und mit einer Skala zum Erkennen der in ihnen jeweils befindlichen Flüssigkeitsmenge versehen sind.

8. Transurethraler Blasenkatheter (1) nach einem der vorangehenden Patentansprüche,
**dadurch gekennzeichnet, dass**
- der Stopfen (5) eine Stufentülle (44) ist.

9. Transurethraler Blasenkatheter (1) nach einem der Patentansprüche 1 bis 8,
**dadurch gekennzeichnet, dass**
- das Leuchtmittel (11) aus einer LED mit einer Abstrahlungscharakteristik von 270° besteht, die über einen Stecker (45) mit O-Ring Dichtung (46) und einen Flüssigkeit abdichtenden Knickschutz (47) mit der Ummantelung des Stromkabels (10) verbunden ist und im eingesteckten Zustand durch eine aufgeschraubte, transparente Kuppel (48) gesichert ist.

10. Transurethraler Blasenkatheter (1) nach einem der vorangehenden Patentansprüche, **dadurch gekennzeichnet, dass**
- als Photosensitizer (8) Dyes, wie Acridine orange, Methyline blue, Rose Bengal, Toludine blue,oder Porphyrine, wie Photofrin, Fotoscan, Aminolevulinic acid, Temoporfin, Talaporfin sodium, oder Chlorine, wie Chlorin e6, oder Furocumarine, wie Psoralen, oder Xanthene, wie Erythrosine, oder Monopene, wie Azulene, eingesetzt werden und die Wellenlänge des Leuchtmittels (11) jeweils an den verwendeten Photosensitizer (8) angepasst ist.

## Claims

1. A transurethral bladder catheter (1) with a main lumen (2) for urinary drainage and at least one free secondary lumen (3) running parallel to the main lumen, and with a two-core biocompatible power cable (10), wherein a switchable and/or controllable power source (12) for controlling a light source (11) is located at the end of the power cable (10) outside the bladder catheter (1),
**characterized in that**
- the free secondary lumen (3) at the distal end (4) of the bladder catheter (1) has an insertable fluid-tight stopper (5) which has both a through channel (6) with a dosing dispenser (7) permanently attached thereto for a photosensitizer (8) and, if required, for further, additional fluids, as well as a fluid-tight, form-fitting and/or material-locking feedthrough (9) for the two-core biocompatible power cable (10), wherein the part of the power cable inserted into the secondary lumen (3), including the light source (11) attached to its end, has the length of the secondary lumen (3).

2. The transurethral bladder catheter (1) according to claim 1, **characterized in that**
- the dosing dispenser (7) contains, in addition to photosensitizers (8), any additional liquids such as rinsing solutions or special medications for various therapies.

3. The transurethral bladder catheter (1) according to any one of claims 1 or 2,
**characterized in that**
- the dosing dispenser (7) consists of an opaque blister-like packaging (13) which has in its interior one or more manually squeezable reservoirs (14) which are connected to a dispensing line (15) leading to the through channel (6), each having duckbill valves (16) at their outlet connections with an opening direction towards the dispensing line (15) and serving for storing and dispensing portioned quantities of the liquid they contain,
- and
- the power source (12) connected to the power cable (10) is located in an airtight and liquid-tight and disinfectant-resistant housing (17) and consists of a power supply (18) with associated externally operable controls (19).

4. The transurethral bladder catheter (1) according to any one of claims 1 or 2,
**characterized in that**
- the dosing dispenser (7) consists of an opaque blister-like packaging (13) which has in its interior one or more machine-squeezable reservoirs (14) which are connected to a dispensing line (15) leading to the through channel (6), each having duckbill valves (16) at their outlet connections with an opening direction towards the dispensing line (15) and serving for storing and dispensing portioned quantities of the liquid they contain,
- the packaging (13) itself contains openings (20) as auxiliary markings for a light barrier (21) required for motion control of the dosing dispenser (7),
and
- an automatic actuator unit (22) controlling the dosing dispenser (7) and the light source (11) is implemented in a cuboid housing (23) whose one end face has a screw terminal (24) consisting of an angle bracket (25) and a locking screw (26) for securing the housing (23), the front surface (27) has a control switch (28) rotatable in 4 positions and an indicator lamp (29), the top surface has a feedthrough for the power cable (10) for connecting the light source (11), a power supply connection (30) equipped with a plug is located on the rear surface opposite the front surface (27), the housing (23) houses at least one light barrier (31) for detecting the openings (20) in the dosing dispenser (7), and two spaced-apart rollers (32) with an electric motor drive (33) arranged parallel to each other in the longitudinal direction of the housing (23) for dispensing bolus doses from the dosing dispenser (7) and a microcontroller (34) controlling these rollers, which simultaneously controls the indicator lamp (29) and determines the switching times of the light source (11)
and
- the housing (23) has a slot-like rectangular opening (35) through which the dosing dispenser (7) located between the two rollers (32) can be pulled in two directions.

5. The transurethral bladder catheter (1) according to claim 1, **characterized in that**
- the power source (12) connected to the power cable (10) is located in an airtight and liquid-tight and disinfectant-resistant housing (17) and consists of a power supply (18) with associated externally operable controls (19)
and
- the dosing dispenser (7) consists of a housing (36) in which a motor-driven spindle drive (37) with an angle step encoder (38) and axially opposing end flanges (39) is located, in the end position of which a fully filled syringe (40) with a photosensitizer (8) can be attached between them.

6. The transurethral bladder catheter (1) according to claim 3 or 5,
**characterized in that**
- the power source (12) has a microcontroller (41) for controlling the illumination duration of the light source (11) over a defined time interval and which simultaneously controls a control lamp (42) indicating the status of the power source (12), a commercially available battery serves as the power supply (18), and an on/off switch (43) is provided as the control (19) which starts the activation of the light source (11) via the microcontroller (41) or interrupts the power supply to the microcontroller (41) to switch off the LED light source (11).

7. The transurethral bladder catheter (1) according to any one of claims 3 or 4,
**characterized in that**
- the reservoirs (14) have different colored markings corresponding to their contents and are equipped with a scale for determining the amount of liquid contained in each of them.

8. The transurethral bladder catheter (1) according to any one of the preceding claims,
**characterized in that**
- the stopper (5) is a stepped nozzle (44).

9. The transurethral bladder catheter (1) according to any one of claims 1 or 8,
**characterized in that**
- the light source (11) consists of an LED with a radiation characteristic of 270°, which is connected to the sheathing of the power cable (10) via a plug (45) with an O-ring seal (46) and a liquid-sealing kink protector (47) and is secured in the plugged-in state by a screwed-on transparent dome (48).

10. The transurethral bladder catheter (1) according to any one of the preceding claims,
**characterized in that**
- dyes, such as acridine orange, methyline blue, rose bengal, toludine blue, or porphyrins, such as photofrin, fotoscan, aminolevulinic acid, temoporfin, talaporfin sodium, or chlorines, such as Chlorine e6, or furocoumarins, such as psoralen, or xanthenes, such as erythrosine, or monopenes, such as azulene, are used as photosensitizers (8) and the wavelength of the light source (11) is adapted to the photosensitizer (8) used.

## Revendications

1. Cathéter vésical transurétral (1) comprenant une lumière principale (2) destinée au drainage de l'urine et au moins une lumière secondaire (3), libre et s'étendant parallèlement à celle-ci, ainsi qu'un câble électrique (10) biocompatible à deux conducteurs, dans lequel une source de courant (12), commutable et/ou pilotable, destinée à commander un moyen d'éclairage (11), est disposée à l'extrémité du câble électrique (10) située à l'extérieur du cathéter vésical (1),
**caractérisé en ce que**
- la lumière secondaire (3) libre comporte, à l'extrémité distale (4) du cathéter vésical (1), un bouchon (5) enfichable étanche aux liquides, lequel possède à la fois un canal de passage (6) muni d'un distributeur-doseur (7) fixé solidairement à celui-ci pour un photosensibilisateur (8) et, le cas échéant, pour d'autres liquides supplémentaires, ainsi qu'un passage (9) étanche aux liquides, réalisé par liaison de forme et/ou par liaison de matière, pour le câble électrique (10) biocompatible à deux conducteurs, dans lequel la partie de celui-ci introduite dans la lumière secondaire (3), y compris le moyen d'éclairage (11) fixé à son extrémité, présente une longueur correspondant à celle de la lumière secondaire (3).

2. Cathéter vésical transurétral (1) selon la revendication 1, **caractérisé en ce que**
- le distributeur-doseur (7) contient, outre des photosensibilisateurs (8), des liquides supplémentaires, tels que des solutions de rinçage ou des médicaments spécifiques destinés à différentes thérapies.

3. Cathéter vésical transurétral (1) selon l'une quelconque des revendications 1 ou 2,
**caractérisé en ce que**
- le distributeur-doseur (7) est constitué d'un emballage (13) de type blister, opaque à la lumière, comportant à l'intérieur un ou plusieurs réservoirs (14), disposés de manière à pouvoir être comprimés manuellement, lesquels sont reliés à une conduite de sortie (15) débouchant dans le canal de passage (6), lesdites connexions situées du côté de la sortie étant chacune pourvues d'une valve à bec (16) avec un sens d'ouverture dirigé vers la conduite de sortie (15), lesdits réservoirs étant destinés au stockage et à la distribution de quantités dosées du liquide qu'ils contiennent respectivement,
- et
- **en ce que** la source de courant (12) reliée au câble électrique (10) est logée dans un boîtier (17) étanche à l'air et aux liquides et résistant aux désinfectants, et est constituée d'une alimentation électrique (18) avec les organes de commande (19) associés, actionnables depuis l'extérieur.

4. Cathéter vésical transurétral (1) selon l'une quelconque des revendications 1 ou 2,
**caractérisé en ce que**
- le distributeur-doseur (7) est constitué d'un emballage (13) de type blister, opaque à la lumière, comportant à l'intérieur un ou plusieurs réservoirs (14), disposés de manière à pouvoir être comprimés mécaniquement, lesquels sont reliés à une conduite de sortie (15) débouchant dans le canal de passage (6), lesdites connexions situées du côté de la sortie étant chacune pourvues d'une valve à bec (16) avec un sens d'ouverture dirigé vers la conduite de sortie (15), lesdits réservoirs étant destinés au stockage et à la distribution de quantités dosées du liquide qu'ils contiennent respectivement,
- l'emballage (13) contient lui-même des perforations (20) servant de repères auxiliaires pour une barrière photoélectrique (21) nécessaire au pilotage du déplacement du distributeur-doseur (7),
et
- une unité d'actionnement (22), commandant automatiquement le distributeur-doseur (7) et le moyen d'éclairage (11), est intégrée dans un boîtier (23) de forme parallélépipédique, dont une face d'extrémité possède un serre-joint à vis (24), constitué d'une équerre (25) et d'une vis de blocage (26), pour la fixation du boîtier (23), dont la face avant (27) présente un commutateur de commande (28) rotatif à quatre positions et un voyant lumineux (29), dont la face supérieure est pourvue d'un passage du câble électrique (10) destiné au raccordement du moyen d'éclairage (11), dont la face arrière, opposée à la face avant (27), est munie d'un raccord d'alimentation électrique (30) équipé d'une fiche, le boîtier (23) contient au moins une une barrière photoélectrique (31) destinée à détecter les perforations (20) présentes dans le distributeur-doseur (7), deux rouleaux (32), espacés l'un de l'autre et orientés parallèlement dans le sens longitudinal du boîtier (23), entraînés par un moteur électrique (33) pour la délivrance de doses en bolus à partir du distributeur-doseur (7), ainsi qu'un microcontrôleur (34) commandant ceux-ci, lequel commande simultanément le voyant lumineux (29) et détermine les temps de commutation du moyen d'éclairage (11)
et
- le boîtier (23) présente une ouverture (35) rectangulaire en forme de fente, à travers laquelle le distributeur-doseur (7), disposé entre les deux rouleaux (32), peut être tiré dans deux directions.

5. Cathéter vésical transurétral (1) selon la revendication 1, **caractérisé en ce que**
- la source de courant (12) reliée au câble électrique (10) est logée dans un boîtier (17) étanche à l'air et aux liquides et résistant aux désinfectants, et est constituée d'une alimentation électrique (18) avec les organes de commande (19) associés, actionnables depuis l'extérieur .
et
- le distributeur-doseur (7) est constitué d'un boîtier (36), dans lequel est disposé un entraînement à vis motorisé (37), muni d'un codeur angulaire incrémental (38) et de brides d'extrémité (39) disposées axialement l'une en regard de l'autre, entre lesquelles, dans leur position d'extrémité, une seringue (40) entièrement remplie d'un photosensibilisateur (8) peut être fixée

6. Cathéter vésical transurétral (1) selon la revendication 3 ou 5,
**caractérisé en ce que**
- la source de courant (12) possède un microcontrôleur (41) destiné à commander la durée d'éclairage du moyen d'éclairage (11) pendant un intervalle de temps défini, celui-ci commandant simultanément un voyant lumineux (42) indiquant l'état de la source de courant (12), une batterie rechargeable du commerce servant d'alimentation électrique (18), et un interrupteur marche/arrêt (43) étant prévu comme organe de commande (19), lequel déclenche, par l'intermédiaire du microcontrôleur (41), l'activation du moyen d'éclairage (11) ou interrompt l'alimentation électrique du microcontrôleur (41) afin d'éteindre le moyen d'éclairage à LED (11).

7. Cathéter vésical transurétral (1) selon l'une quelconque des revendications 3 ou 4,
**caractérisé en ce que**
- les réservoirs (14) présentent des marquages de couleurs différentes correspondant à leur contenu et sont pourvus d'une échelle graduée permettant de déterminer la quantité de liquide contenue dans chacun d'eux.

8. Cathéter vésical transurétral (1) selon l'une quelconque des revendications précédentes,
**caractérisé en ce que**
- le bouchon (5) est un embout cannelé étagé (44).

9. Cathéter vésical transurétral (1) selon l'une quelconque des revendications 1 ou 8,
**caractérisé en ce que**
- le moyen d'éclairage (11) est constitué d'une LED présentant une caractéristique de rayonnement de 270°, laquelle est reliée à la gaine du câble électrique (10) par l'intermédiaire d'un connecteur (45) muni d'un joint torique (46) et d'un dispositif anti-pliure (47) assurant l'étanchéité aux liquides, et est, à l'état enfiché, maintenue en place par un dôme transparent (48) vissé.

10. Cathéter vésical transurétral (1) selon l'une quelconque des revendications précédentes,
**caractérisé en ce que**
- des colorants, tels que l'acridine orange, le bleu de méthylène, le Rose Bengale, le bleu de toluidine, ou des porphyrines, telles que Photofrin, Fotoscan, l'acide aminolévulinique, le témoporfine, le talaporfine sodique, ou des chlorines, telles que la chlorine e6, ou des furocoumarines, telles que le psoralène, ou des xanthènes, tels que l'érythrosine, ou des monoterpènes, tels que les azulènes, sont utilisés en tant que photosensibilisateur (8), et la longueur d'onde du moyen d'éclairage (11) est adaptée à chaque photosensibilisateur (8) utilisé.
